# EUROPEAN PATENT APPLICATION

(11) **EP 4 631 447 A1**
(43) Date of publication of application: **15.10.2025**
(21) Application number: 24741112.7
(22) Date of filing: 02.01.2024
(51) Int. Cl.: A61B 17/34

(54) **BALLOON PUNCTURE DEVICE**

(30) Priority: 09.01.2023 CN 202320064164 U
(71) Applicant: Surgsci Medical Ltd., Shenzhen, Guangdong 518101 (CN)
(72) Inventor: LV, Xiaobo, Shenzhen, Guangdong 518101 (CN)
(74) Representative: Prinz & Partner mbB
(86) International application number: PCT/CN2024/070141
(87) International publication number: WO 2024/149113

(57) **Abstract**

A balloon trocar includes a cannula assembly and a balloon tube assembly. The cannula assembly includes a base, a cannula extending along the axis of the base and integrally formed with the base, and an insufflation valve body disposed on the base, and the insufflation valve body communicates with a gas inlet duct. The balloon tube assembly is securely sleeved outside of the cannula and located between the base and a bottom end of the cannula and includes a balloon tube, a valve sleeve, and a balloon that are integrally formed from an elastic material, and the valve sleeve and the balloon are disposed at positions close to two ends of the balloon tube respectively and can communicate with each other through a fluid passage disposed on the inner sidewall of the balloon tube. **In** the balloon trocar, the cannula and the base are convenient and easy to process. The balloon trocar has a high production yield and a low cost.

## Description

The present application claims priority to Chinese Patent Application No. 202320064164.5, entitled "BALLOON TROCAR" and filed with the China National Intellectual Property Administration (CNIPA) on Jan. 09, 2023, the disclosure of which is incorporated herein by reference in its entirety.

### TECHNICAL FIELD

The present application belongs to the technical field of medical instruments and, more specifically, to a balloon trocar.

### BACKGROUND

As a commonly used tool in laparoscopic surgery, a balloon trocar is used for establishing a passage for surgical instruments to enter the human body and maintaining the pneumoperitoneum required for surgery.

The balloon trocar at least includes a cannula, a base, and a balloon tube assembly.

Specifically, the cannula is not only provided with a valve sleeve for assembling a check valve but also formed with a flow guiding groove communicating with the valve sleeve; the balloon tube assembly includes a balloon tube sleeved outside of the cannula and a balloon provided on the balloon tube, and when the balloon tube is sleeved on the cannula, the flow guiding groove on the cannula can communicate with the balloon. Therefore, gas can be inflated into the flow guiding groove on the cannula by assembling the check valve on the valve sleeve, thereby expanding the balloon.

However, in the related art, generally, the cannula and the base are integrally formed through an injection mold. Since the flow guiding groove on the cannula communicates with the valve sleeve, the design of a fluid channel in the cannula is caused to be complex, which makes the processing requirement on the injection mold high. As a result, on the one hand, the injection mold is difficult to manufacture, resulting in a high production cost, and on the other hand, the yield of the cannula and the base is also low, further increasing the manufacturing cost of the balloon trocar.

### SUMMARY

### Technical Problem

An object of embodiments of the present application is to provide a balloon trocar to solve the technical problem of difficulty in manufacturing the cannula and the base in the balloon trocar in the existing art.

### Technical Solution

To achieve the preceding object, the technical solution adopted in the embodiments of the present application is to provide a balloon trocar. The balloon trocar includes a cannula assembly and a balloon tube assembly.

The cannula assembly includes a base, a cannula extending along an axis of the base and integrally formed with the base, and an insufflation valve body disposed on the base, where the insufflation valve body communicates with a gas inlet duct.

The balloon tube assembly is securely sleeved outside of the cannula and located between the base and a bottom end of the cannula, and the balloon tube assembly includes a balloon tube, a valve sleeve, and a balloon that are integrally formed from an elastic material, where the valve sleeve and the balloon are disposed at positions close to two ends of the balloon tube respectively, and the valve sleeve and the balloon are capable of communicating with each other through a fluid passage disposed on an inner sidewall of the balloon tube.

Optionally, the balloon tube assembly is integrally formed from transparent or translucent elastic silica gel.

Optionally, a wall thickness of the valve sleeve is larger than a wall thickness of the balloon tube, the valve sleeve is capable of housing an end of a check valve, and a fluid passing through the fluid passage via the check valve is allowed to flow into the balloon.

Optionally, the base further includes an inner cannula base and an outer cannula base, and the inner cannula base, the outer cannula base, the insufflation valve body, and the gas inlet duct are integrally formed.

Optionally, the two ends of the balloon tube are securely and sealably connected to the cannula respectively through hot melting or gluing.

Optionally, an annular groove is formed at each of positions in which an outer surface of the cannula is securely and sealably connected to the balloon tube.

Optionally, an annular protruding region is formed between the annular groove of the cannula and a bottom of the base, and a region spaced between an end of the balloon tube close to the valve sleeve and the valve sleeve includes one extended tubular step region, where the tubular step region directly covers and is sealably secured to the annular protruding region.

Optionally, a flat portion is formed on a circumferential surface of the cannula along an axis of the cannula, where the flat portion is located directly below the insufflation valve body, the flat portion extends from a position of the cannula covered by the valve sleeve to a position of the cannula covered by the balloon, and the fluid passage covers the flat portion in parallel.

Optionally, the fluid passage disposed on the inner sidewall of the balloon tube along an axis of the balloon tube is a sealed tubular passage, or the inner sidewall of the balloon tube is formed with a flow guiding groove opened toward the cannula, wherein the flow guiding groove is combined with a surface of the cannula to form a complete fluid passage.

Optionally, the balloon trocar further includes a flexible securing outer tube and a limiter slidably disposed on the securing outer tube, where the securing outer tube is sleeved on an outer sidewall of a portion of the balloon tube that is located between the balloon and the valve sleeve.

### Beneficial Effect

The balloon trocar provided in the embodiments of the present application has at least the following beneficial effects.

Compared with the arrangement of disposing the valve sleeve on the cannula in the related art, the valve sleeve is disposed on the balloon tube, and the balloon tube, the balloon, and the valve sleeve are integrally formed. In this arrangement in the present application, there is no need to dispose a complex fluid channel inside the cannula assembly, thereby reducing the processing difficulty of the cannula assembly and simplifying the complexity of a mold required for processing the cannula assembly. Moreover, the base and the cannula in the cannula assembly are also integrally formed, which can reduce the assembly error and the generation of a gap between the base and the cannula, thereby helping reduce the production cost and improve the production yield.

### BRIEF DESCRIPTION OF DRAWINGS

To illustrate the technical solution in the embodiments of the present application more clearly, drawings used in the description of the embodiments or the existing art are briefly described below. Apparently, the drawings described below illustrate only some embodiments of the present application, and those of ordinary skill in the art may obtain other drawings based on the drawings described below on the premise that no creative work is done.
FIG. 1 is a perspective view of a balloon trocar according to some embodiments of the present application.
FIG. 2 is an exploded view of a balloon trocar according to some embodiments of the present application.
FIG. 3 is a sectional view of a cannula assembly according to some embodiments of the present application.
FIG. 4 is a front view of a cannula assembly according to some embodiments of the present application.
FIG. 5 is a front view of a balloon tube assembly according to some embodiments of the present application.
FIG. 6 is a sectional view of a balloon tube assembly according to some embodiments of the present application.

### Reference list

- 100: cannula assembly
- 110: cannula
- 111: glue groove
- 112: flat portion
- 113: annular protruding region
- 120: base
- 121: insufflation valve body
- 122: gas inlet duct
- 123: inner cannula base
- 124: outer cannula base
- 130: puncture head
- 200: balloon tube assembly
- 210: balloon tube
- 211: flow guiding groove
- 212: tubular step region
- 220: valve sleeve
- 230: balloon
- 231: reinforcing rib
- 300: securing tube
- 400: limiter
- 500: check valve

### DETAILED DESCRIPTION

The present application is further described in detail in conjunction with the drawings and embodiments, from which the technical problems to be solved, the technical solutions, and the beneficial effects of the present application are more apparent.

It is to be understood that the embodiments described herein are intended to explain and not to limit the present application.

It is to be noted that when an element is described as being "secured to" or "disposed on" another element, the element may be directly on the particular element or indirectly on the particular element.

When an element is described as being "connected to" another element, the element may be directly connected to the particular element or indirectly connected to the particular element.

It is to be understood that orientations or position relations indicated by terms such as "length", "width", "above", "below", "front", "rear", "left", "right", "vertical", "horizontal", "top", "bottom", "inside", "outside" and the like are based on orientations or position relations shown in the drawings. These orientations or position relations are intended only to facilitate and simplify the description of the present application, and not to indicate or imply that a device or element referred to must have such specific orientations or must be configured or operated in such specific orientations. Thus, these orientations or position relations are not to be construed as limiting the present application.

Moreover, terms such as "first" and "second" are used only for the purpose of description and are not to be construed as indicating or implying relative importance or implicitly indicating the number of technical features indicated. Thus, a feature defined as a "first" feature or a "second" feature may explicitly or implicitly include one or more of such features.

In the description of the present application, the term "plurality" is defined as two or more, unless otherwise expressly specified and limited.

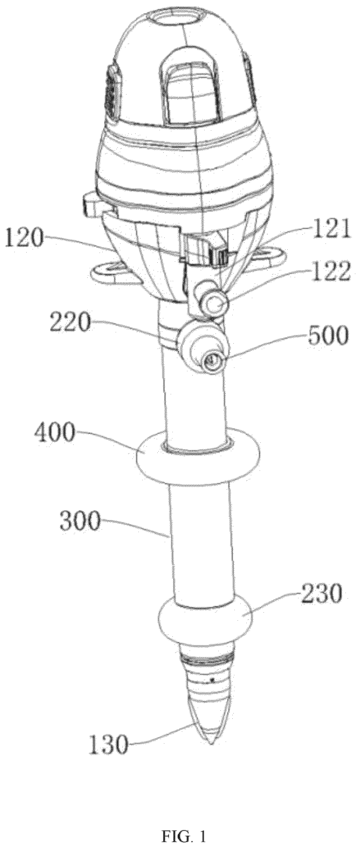

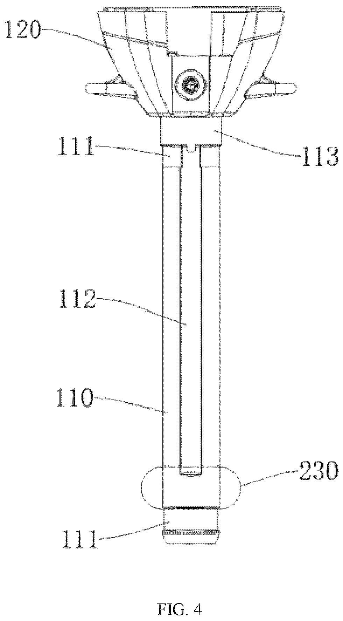

Referring to FIGS. 1 to 6, a balloon trocar provided in the embodiments of the present application is described below.

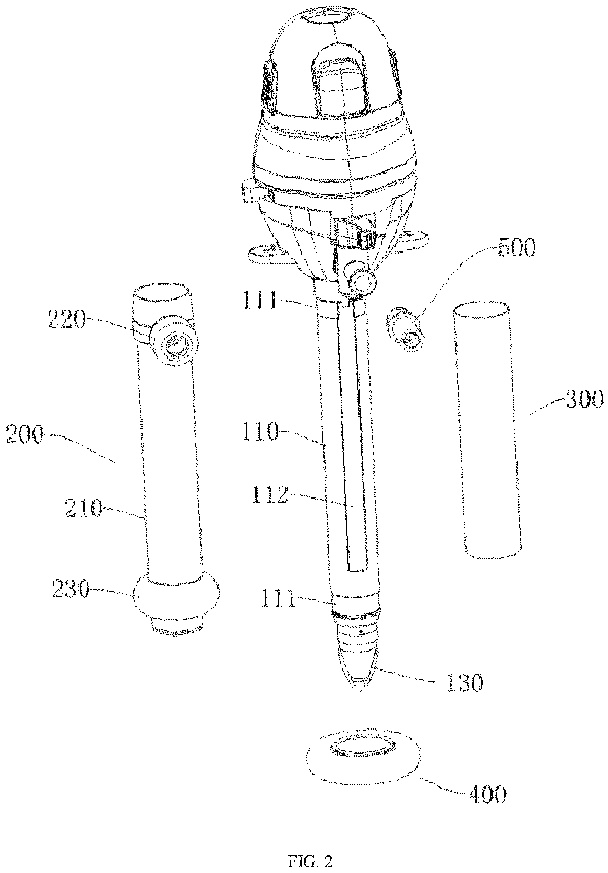

Referring to FIGS. 1 and 2, the balloon trocar described in the present application includes a cannula assembly 100 and a balloon tube assembly 200.

Specifically, the cannula assembly 100 includes a cannula 110 and a base 120.

The cannula 110 is disposed at the bottom of the base 120. The cannula 110 is hollow, and the hollow interior of the cannula 110 may be used for a puncture rod provided with a puncture head 130 to penetrate through.

When the puncture rod penetrates through the hollow interior of the cannula 110, the puncture head 130 on the puncture rod is used for puncturing the abdominal wall to place a balloon 230 on the inner side of the abdominal wall. After the balloon 230 is placed and inflated, the puncture rod is pulled out from the cannula 110.

The base 120 is provided with an insufflation valve body 121, and the insufflation valve body 121 communicates with a gas inlet duct 122. The gas inlet duct 122 is used for connecting the air nozzle of a pneumoperitoneum machine. After the balloon 230 is placed and inflated, and the puncture rod has been pulled out from the cannula 110, the pneumoperitoneum machine blows gas toward the air nozzle, and the gas enters the cannula 110 after passing through the insufflation valve body 121 and flows into the abdominal wall through a bottom outlet of the cannula 110 to form pneumoperitoneum.

Furthermore, the cannula 110 and the base 120 are integrally formed. In this manner, the production yield of the cannula assembly 100 can be improved, the generation of an assembly gap between the cannula 110 and the base 120 can be reduced, and the airtightness between the cannula 110 and the base 120 can be improved. When the pneumoperitoneum machine inflates the insufflation valve body 121, the laparoscopic surgery can be prevented from being affected due to the leakage of the gas at the connection gap between the cannula 110 and the base 120.

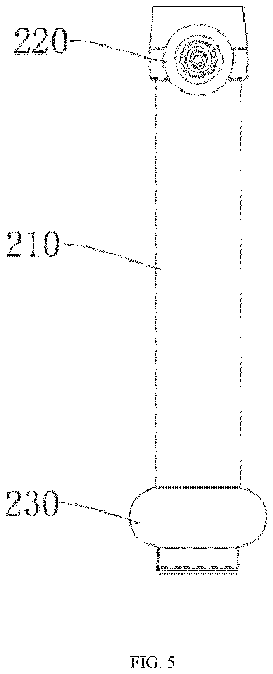

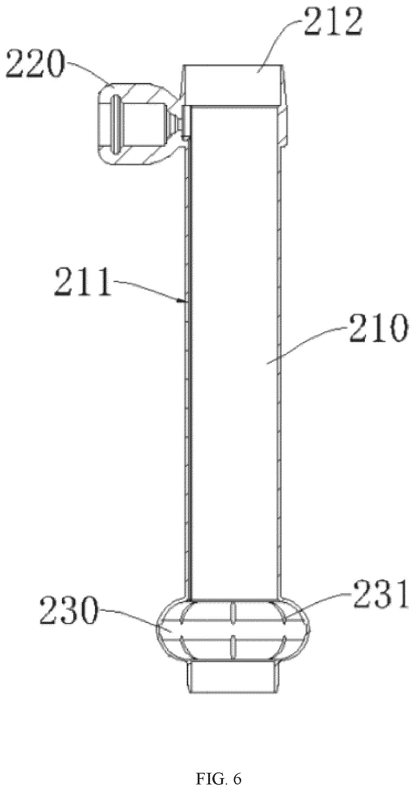

Referring to FIGS. 1 and 2, the balloon tube assembly 200 is sleeved outside of the cannula 110 and located between the base 120 and a bottom end of the cannula 110. Specifically, the balloon tube assembly 200 includes a balloon tube 210, a valve sleeve 220, and the balloon 230 that are integrally formed from an elastic material. Moreover, referring to FIGS. 5 and 6, the valve sleeve 220 and the balloon 230 are formed at positions on the balloon tube 210 close to two ends of the balloon tube 210 respectively.

In this manner, the balloon tube 210, the valve sleeve 220, and the balloon 230 are integrally formed from the elastic material. Compared with the arrangement of disposing the valve sleeve 220 on the cannula assembly 100 in the related art, there is no need to dispose a complex fluid channel in the cannula assembly 100, which can simplify the injection molding process, improve the yield, and simplify the injection mold, thereby reducing the production cost.

Furthermore, in the balloon tube assembly 200, the inner sidewall of the balloon tube 210 is provided with a fluid passage, and the fluid passage communicates with the balloon 230 and the valve sleeve 220 so that a fluid, such as gas or liquid, can flow between the valve sleeve 220 and the balloon 230.

The valve sleeve 220 is used for externally connecting a check valve 500. When the cannula 110 is inserted into the abdominal wall, and the balloon 230 is located on the inner side of the abdominal wall, a fluid filling and discharging apparatus (such as a syringe) can fill the balloon 230 with the fluid (such as the gas or liquid) through the check valve 500, thereby expanding the balloon 230 to seal an opening on the abdominal wall.

In conclusion, compared with the related art, the balloon trocar of the embodiments of the present application can simplify the production process, improve the production yield, and thereby reduce the production cost.

In some embodiments of the present application, the balloon tube assembly 200 is made of a transparent or translucent elastic material, such as silica gel.

With this arrangement, for one aspect, compared with the arrangement of the balloon tube 210 and the balloon 230 made of a polypropylene (PP) blow molding material in the related art, the balloon tube assembly 200 is made of silica gel so that the balloon tube 210 and the balloon 230 have better elasticity, and the balloon 230 is not easy to rupture when the balloon 230 is filled with the fluid, thereby improving the use safety; for another aspect, the inner diameter of the balloon tube 210 can be made slightly smaller than the outer diameter of the cannula 110, and when the balloon tube 210 is sleeved outside of the cannula 110, the inner sidewall of the balloon tube 210 can be fitted to the outer sidewall of the cannula 110 tightly, thereby securing the balloon tube assembly 200 to the cannula 110 to prevent the balloon tube 210 from sliding relative to the cannula 110 during the laparoscopic surgery.

Furthermore, in the balloon tube assembly 200, the wall thickness of the valve sleeve 220 is larger than the wall thickness of the balloon tube 210.

In this manner, the valve sleeve 220 has sufficient strength and elastic force, and when the check valve 500 is assembled on the valve sleeve 220, a mounting chamber inside the valve sleeve 220 can undergo slight elastic deformation to tightly wrap the check valve 500, thereby avoiding the leakage of the fluid due to a gap between the check valve 500 in the valve sleeve 220 and the sidewall of the mounting chamber in the valve sleeve 220.

Furthermore, referring to FIG. 6, the inner sidewall of the balloon 230 is formed with reinforcing ribs 231. In this manner, the structural strength of the balloon 230 after inflation can be enhanced, thereby helping increase the reliability of the balloon 230 in sealing the opening on the abdominal wall and improve the surgical safety.

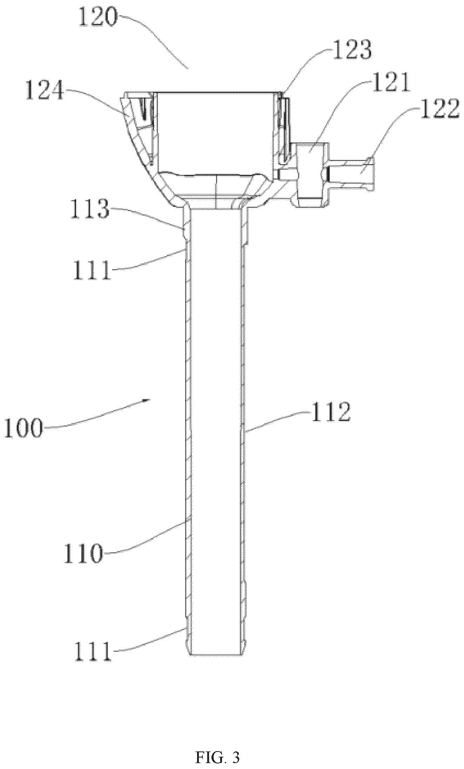

Referring to FIG. 3, in some embodiments of the present application, the base 120 further includes an inner cannula base 123 and an outer cannula base 124, and the inner cannula base 123, the outer cannula base 124, the insufflation valve body 121, and the gas inlet duct 122 are integrally formed.

It is to be understood that the inner cannula base 123 is used for a handle of the puncture rod to insert into, and a gap between the inner cannula base 123 and the outer cannula base 124 is used for securing the handle of the puncture rod.

The inner cannula base 123, the outer cannula base 124, the insufflation valve body 121, and the gas inlet duct 122 are integrally formed so that the manufacturing difficulty of the cannula assembly 100 can be reduced while conventional functions of components on the base 120 are fulfilled. Moreover, since the components on the base 120 are integrally formed, and the base 120 is integrally formed with the cannula 110, the generation of assembly gaps between components on the cannula assembly 100 can be avoided, thereby helping improve the airtightness and reliability of the cannula assembly 100.

Referring to FIGS. 2 and 4 to 6, it is to be understood that, as mentioned in the preceding, the balloon 230 and the valve sleeve 220 are disposed at the positions on the balloon tube 210 close to the two ends of the balloon tube 210 respectively. Thus, a region between one end of the balloon tube 210 close to the balloon 230 and the balloon 230 is defined as a first securing region, and a region between the other end of the balloon tube 210 close to the valve sleeve 220 and the valve sleeve 220 is defined as a second securing region.

In some embodiments of the present application, the first securing region and the second securing region on the balloon tube 210 are both securely and sealably connected to the cannula 110.

For example, the sealing connection may be hot melting or gluing.

It is to be understood that, in the hot-melt connection mode, the balloon tube assembly 200 may be made of a hot-melt elastic material.

In the gluing connection mode, the balloon tube assembly 200 is only made of an elastic material. During assembly, specifically, the balloon tube assembly 200 is first sleeved on the cannula 110, the first securing region and the second securing region on the balloon tube 210 are temporarily separated from predetermined positions on the cannula 110, then glue is applied to the sleeved positions on the cannula 110 corresponding to the first securing region and the second securing region, and then the first securing region and the second securing region on the balloon tube 210 cover corresponding positions on the cannula 110 where the glue is applied, so as to enable the gluing connection.

The balloon tube 210 is configured in this manner. Since the first securing region and the second securing region on the balloon tube 210 are secured and sealed, when the balloon 230 is filled with the fluid, the fluid does not leak from the two ends of the balloon tube 210, thereby improving the safety of using the balloon trocar.

Referring to FIGS. 2 to 4, in some embodiments of the present application, to further facilitate the coating of the glue, an annular groove 111 is formed at each of positions in which the outer surface of the cannula 110 is securely and sealably connected to the balloon tube 210. The annular groove 111 can be completely covered by the balloon tube 210 and is used for filling the glue to secure the balloon tube 210.

That is, the outer surface of the cannula 110 is formed with two annular grooves 111, and the annular grooves 111 extend around the circumferential direction of the cannula 110. One annular groove 111 corresponds to the first securing region on the balloon tube 210, and the other annular groove 111 corresponds to the second securing region on the balloon tube 210.

With this arrangement, the annular grooves 111 can be staggered with the balloon 230 at axial positions so that when the balloon 230 is filled with the fluid, the fluid cannot leak from the annular grooves 111, thereby improving the safety of using the balloon trocar.

Specifically, after the balloon tube 210 is sleeved on the cannula 110, the first securing region and the second securing region on the balloon tube 210 are temporarily separated from the predetermined positions on the cannula 110, then the annular grooves 111 are filled with the glue, and then the first securing region and the second securing region on the balloon tube 210 cover the annular grooves 111, thereby enabling the gluing connection between the cannula 110 and the balloon tube 210.

It is to be understood that the annular grooves 111 and the cannula 110 are integrally formed.

Referring to FIGS. 3, 4, and 6, in some embodiments of the present application, to facilitate the assembly of the balloon tube assembly 200, the cannula assembly 100 and the balloon tube 210 are configured as follows: an annular protruding region 113 is formed between an annular groove 111 on the cannula 110 close to the base 120 and the bottom of the base 120, and a tubular step region 212 is formed in a region spaced between an end of the balloon tube 210 close to the valve sleeve 220 and the valve sleeve 220; the tubular step region 212 is used for covering and being sealably secured to the annular protruding region 113.

It is to be understood that in the case where the cannula 110 is formed with the annular protruding region 113, the annular groove 111 corresponding to the second securing region is disposed on the bottom side of the annular protruding region 113.

The cannula 110 is formed with the annular protruding region 113, and the balloon tube 210 is formed with the tubular step region 212 so that the positioning of the balloon tube 210 is facilitated when the balloon tube 210 is sleeved on the cannula 110, thereby helping improve the assembly efficiency.

Similarly, it is to be understood that the annular protruding region 113 and the cannula 110 are also integrally formed.

Referring to FIGS. 2 to 4, in some embodiments of the present application, a flat portion 112 is formed on the circumferential surface of the cannula 110 and extends along the axial direction of the cannula 110.

The flat portion 112 is located directly below the insufflation valve body 121. The flat portion 112 extends from a position on the cannula 110 covered by the valve sleeve 220 to a position on the cannula 110 covered by the balloon 230, and the fluid passage covers the flat portion 112 in parallel.

At least the following effects are achieved by forming the flat portion 112 on the circumferential surface of the cannula 110.

First, it is convenient to use the balloon trocar during surgery. After the balloon tube assembly 200 is secured to the cannula 110, the valve sleeve 220 and the insufflation valve body 121 are located in the same straight line direction. During the surgical use of the balloon trocar, the fluid filling and discharging apparatus connected to the check valve 500 and a gas tube of the pneumoperitoneum machine connected to the insufflation valve body 121 are both located on the same side of the balloon trocar, which can facilitate the operation of medical personnel.

Second, this can also play a positioning role in the sleeving of the balloon tube assembly 200 to improve the assembly efficiency of the balloon trocar. When a sleeving operation is performed on the balloon tube assembly 200, and when the fluid passage on the balloon tube 210 covers the flat portion 112 in parallel, it means that the secure sleeving of the balloon tube assembly 200 is completed so that the accuracy of securely sleeving the balloon tube assembly 200 is easy to ensure.

It is to be understood that the preceding fluid passage is disposed on the inner sidewall of the balloon tube 210, and there may be various embodiments.

For example, in some embodiments, a sealed tubular passage is disposed on the inner sidewall of the balloon tube 210 along the axial direction of the balloon tube 210.

It is to be understood that the arrangement of the fluid passage in this embodiment is applicable regardless of whether the flat portion 112 is formed on the cannula 110.

For another example, in some other embodiments, the inner sidewall of the balloon tube 210 is formed with a flow guiding groove 211 opened toward the cannula 110, and the flow guiding groove 211 is combined with the surface of the cannula 110 to form a fluid passage.

Similarly, the arrangement of the flow guiding groove 211 in this embodiment is applicable regardless of whether the flat portion 112 is formed on the surface of the cannula 110.

Specifically, in some embodiments, in the arrangement in which the flat portion 112 is not formed on the surface of the cannula 110, the flow guiding groove 211 is combined with the surface of the cannula 110 to form the fluid passage.

In some other embodiments, that is, in the arrangement in which the flat portion 112 is disposed on the surface of the cannula 110, the flow guiding groove 211 is combined with the surface of the flat portion 112 on the cannula 110 to form the fluid passage. It is to be understood that in this embodiment, the bottom of the flat portion 112 is spaced a certain distance from the annular groove 111, and the distance from the bottom of the flat portion 112 to the annular groove 111 is smaller than the height of the balloon 230 in the axial direction of the balloon tube 210. In this manner, when the flow guiding groove 211 is combined with the surface of the flat portion 112 on the cannula 110 to form the fluid passage, the fluid passage does not communicate with the annular groove 111, thereby further avoiding fluid leakage.

Furthermore, in this embodiment, the flow guiding groove 211 may have a variety of cross sections, such as a semicircular cross section, a rectangular cross section, a triangular cross section, or any other shaped cross section, as long as the flow guiding groove 211 and the surface of the flat portion 112 can form the fluid passage after the balloon tube 210 is sleeved on the cannula 110.

Referring to FIGS. 1 and 2, in some embodiments of the present application, the balloon trocar further includes a flexible securing tube 300, and the securing tube 300 is sleeved on the outer sidewall of a portion of the balloon tube 210 that is located between the balloon 230 and the valve sleeve 220.

It is to be understood that the securing tube 300 is made of a heat-shrink tubing material and is foldable but not elastic.

After the balloon tube 210 is sleeved on the cannula 110, the heat-shrinkable securing tube 300 is sleeved on the outer sidewall of a portion of the balloon tube 210 that is located between the balloon 230 and the valve sleeve 220.

In this manner, the stability of the axial position of the balloon tube 210 on the cannula 110 can be further improved.

Moreover, in the preceding arrangement in which the fluid passage is formed between the flow guiding groove 211 and the outer surface of the cannula 110, a portion of the balloon tube 210 other than the portion that forms the fluid passage together with the outer surface of the cannula 110 can be closely fitted to the cannula 110 so that the fluid can flow to the balloon 230 as much as possible, thereby improving the inflation efficiency of the balloon 230.

In addition, it is to be understood that in this embodiment, the outer sidewall of the balloon tube 210 that is sleeved with the securing tube 300 is roughened so that the friction between the securing tube 300 and the balloon tube 210 can be increased to further improve the securing effect of the securing tube 300 on the balloon tube 210.

Moreover, the balloon trocar further includes a limiter 400, and the limiter 400 is sleeved outside of the securing tube 300 and can slide along the axial direction of the securing tube 300.

The limiter 400 is disposed and can slide along the axial direction of the balloon tube 210 so that before the balloon trocar is inserted into the abdominal wall, the limiter 400 can be slid to an axial position farther away from the balloon 230. Moreover, after the insertion position of the balloon trocar is secured, and the balloon 230 is inflated, the insertion state of the balloon trocar can be secured by pushing the limiter 400 to slide along the axial direction of the balloon tube 210 until the limiter 400 abuts against the abdominal wall, thereby preventing the surgical safety from being affected due to the movement of the balloon trocar during surgery and also improving the airtightness of the insertion position of the balloon trocar on the abdominal wall to a certain extent.

The preceding are preferred embodiments of the present application and not intended to limit the present application. Any modifications, equivalent substitutions, improvements, and the like made within the spirit and principle of the present application are within the scope of the present application.

## Claims

1. A balloon trocar, comprising:
a cannula assembly, wherein the cannula assembly comprises a base, a cannula extending along an axis of the base and integrally formed with the base, and an insufflation valve body disposed on the base, wherein the insufflation valve body communicates with a gas inlet duct; and
a balloon tube assembly, wherein the balloon tube assembly is securely sleeved outside of the cannula and located between the base and a bottom end of the cannula, and the balloon tube assembly comprises a balloon tube, a valve sleeve, and a balloon that are integrally formed from an elastic material, wherein the valve sleeve and the balloon are disposed at positions close to two ends of the balloon tube respectively, and the valve sleeve and the balloon are capable of communicating with each other through a fluid passage disposed on an inner sidewall of the balloon tube.

2. The balloon trocar of claim 1, wherein the balloon tube assembly is integrally formed from transparent or translucent elastic silica gel.

3. The balloon trocar of claim 1, wherein a wall thickness of the valve sleeve is larger than a wall thickness of the balloon tube, the valve sleeve is capable of housing an end of a check valve, and a fluid passing through the fluid passage via the check valve is allowed to flow into the balloon.

4. The balloon trocar of claim 1, wherein the base further comprises an inner cannula base and an outer cannula base, and the inner cannula base, the outer cannula base, the insufflation valve body, and the gas inlet duct are integrally formed.

5. The balloon trocar of claim 1, wherein the two ends of the balloon tube are both securely and sealably connected to the cannula through hot melting or gluing.

6. The balloon trocar of claim 5, wherein an annular groove is formed at each of positions in which an outer surface of the cannula is securely and sealably connected to the balloon tube.

7. The balloon trocar of claim 6, wherein an annular protruding region is formed between the annular groove of the cannula and a bottom of the base, and a region spaced between an end of the balloon tube close to the valve sleeve and the valve sleeve comprises one extended tubular step region, wherein the tubular step region directly covers and is sealably secured to the annular protruding region.

8. The balloon trocar of claim 1, wherein a flat portion is formed on a circumferential surface of the cannula along an axis of the cannula, wherein the flat portion is located directly below the insufflation valve body, the flat portion extends from a position of the cannula covered by the valve sleeve to a position of the cannula covered by the balloon, and the fluid passage covers the flat portion in parallel.

9. The balloon trocar of claim 1 or 8, wherein the fluid passage disposed on the inner sidewall of the balloon tube along an axis of the balloon tube is a sealed tubular passage, or the inner sidewall of the balloon tube is formed with a flow guiding groove opened toward the cannula, wherein the flow guiding groove is combined with a surface of the cannula to form a complete fluid passage.

10. The balloon trocar of claim 1, further comprising a flexible securing outer tube and a limiter slidably disposed on the securing outer tube, wherein the securing outer tube is sleeved on an outer sidewall of a portion of the balloon tube that is located between the balloon and the valve sleeve.
